(19) Europäisches Patentamt
European Patent Office
Office européen des brevets

(11) EP 3 305 278 A1

(12) **EUROPEAN PATENT APPLICATION**
published in accordance with Art. 153(4) EPC

(43) Date of publication:
**11.04.2018 Bulletin 2018/15**

(21) Application number: **16803166.4**

(22) Date of filing: **25.05.2016**

(51) Int Cl.:
***A61K 8/81*** *(2006.01)* ***A61Q 3/02*** *(2006.01)*

(86) International application number:
**PCT/JP2016/065441**

(87) International publication number:
**WO 2016/194731 (08.12.2016 Gazette 2016/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.05.2015 JP 2015109327**

(71) Applicant: **FUJIFILM Corporation**
**Tokyo 106-8620 (JP)**

(72) Inventors:
- **ISHIJI, Yohei**
**Haibara-gun**
**Shizuoka 421-0396 (JP)**
- **OOHASHI, Hidekazu**
**Haibara-gun**
**Shizuoka 421-0396 (JP)**

(74) Representative: **Hoffmann Eitle**
**Patent- und Rechtsanwälte PartmbB**
**Arabellastraße 30**
**81925 München (DE)**

# (54) NAIL COSMETIC AND NAIL ART KIT

(57) An object of the present invention is to provide a nail cosmetic material having excellent adhesiveness and peelability, and a nail art kit containing the nail cosmetic material.

The nail cosmetic material of the present invention including: diacetone acrylamide as a component 1; and at least one compound selected from the group consisting of isobornyl methacrylate, hydroxyethyl acrylamide, methacrylamide, dimethyl acrylamide, diethyl acrylamide, acryloyl morpholine, and butoxy diethylene glycol methacrylate, as a component 2.

EP 3 305 278 A1

## Description

The present invention relates to a nail cosmetic material and a nail art kit.

In recent years, the popularity of nail art that designs nails of the hands or feet is increasing, and a technology for forming artificial nails (fake nails, nail tips, and the like) made of synthetic resins on nails (natural nails) has been developed (refer to JP2004-275736A). Particularly, recently, a nail decoration called an artificial nail, which is used by applying a gel-like decorative curable composition containing a urethane resin and a photopolymerizable monomer on the nail followed by curing the composition through irradiation of the composition with ultraviolet rays, is attracting attention due to reasons that the nail decoration provides a clear finish, is long-wearing and highly adhesive to the nail, and is odorless as in a case of an acrylic resin (JP2002-505915U).

A composition in which acrylamide monomer being a liquid at 25°C is used is known as such a gel-like decorative curable composition (JP4981184B).

An object of the present invention is to provide a nail cosmetic material having excellent adhesiveness and peelability, and a nail art kit containing the nail cosmetic material.

The object is achieved by means described in the following <1> or <12>. The object will be shown below together with <2> to <11> which are preferred embodiments.

<1> A nail cosmetic material comprising: diacetone acrylamide as a component 1; and at least one compound selected from the group consisting of isobornyl methacrylate, hydroxyethyl acrylamide, methacrylamide, dimethyl acrylamide, diethyl acrylamide, acryloyl morpholine, and butoxy diethylene glycol methacrylate, as a component 2.
<2> The nail cosmetic material according to <1>, in which the component 2 includes at least two compounds selected from the group consisting of isobornyl methacrylate, hydroxyethyl acrylamide, methacrylamide, dimethyl acrylamide, diethyl acrylamide, acryloyl morpholine, and butoxy diethylene glycol methacrylate.
<3> The nail cosmetic material according to <1>, in which the component 2 includes isobornyl methacrylate; and at least one compound selected from the group consisting of hydroxyethyl acrylamide, methacrylamide, and dimethyl acrylamide.
<4> The nail cosmetic material according to any one of <1> to <3>, further comprising: a photopolymerization initiator.
<5> The nail cosmetic material according to any one of <1> to <4>, further comprising: a polymer and/or an oligomer.
<6> The nail cosmetic material according to <5>, in which the polymer and/or the oligomer have a urethane bond.
<7> The nail cosmetic material according to <5> or <6>, in which the polymer and/or the oligomer have a (meth)acrylic group.
<8> The nail cosmetic material according to any one of <5> to <7>, in which the total content of the polymer and the oligomer is 10 to 60 parts by mass with respect to 100 parts by mass of the nail cosmetic material.
<9> The nail cosmetic material according to any one of <1> to <8>, in which the content of the component 1 is 1 to 50 parts by mass with respect to 100 parts by mass of the nail cosmetic material.
<10> The nail cosmetic material according to any one of <1> to <9>, in which the total content of the components 2 is 5 to 80 parts by mass with respect to 100 parts by mass of the nail cosmetic material.
<11> The nail cosmetic material according to any one of <1> to <10>, further comprising: an organic solvent.
<12> A nail art kit comprising:

the nail cosmetic material according to any one of <1> to <11>.

According to the present invention, it is possible to provide a nail cosmetic material having excellent adhesiveness and peelability, and a nail art kit containing the nail cosmetic material.

Hereinafter, the present invention will be described in detail.

In the present specification, the description "xx to yy" represents a numerical range including xx and yy.

"(Meth)acrylamide" or the like has the same meaning as "acrylamide and/or methacrylamide" or the like, and the same applies to others in the following.

In addition, in the present invention, "mass%" has the same meaning as that of wt%" and "parts by mass" has the same meaning as that of "parts by weight".

In addition, in the present invention, a combination of two or more preferable embodiments is a more preferable embodiment.

In the present specification, regarding description of a group in a compound represented by a formula, in a case where there is no description as to whether the group is substituted or unsubstituted, the group also contains a group having a substituent as well as an unsubstituted group unless otherwise specified in a case where the group can further have the substituent. For example, in a formula, in a case where there is a description that "R represents an alkyl group, an aryl group, or a heterocyclic group", the description means that "R represents an unsubstituted alkyl group, a substituted alkyl group, an unsubstituted aryl group, a substituted aryl group, an unsubstituted heterocyclic group, or a

substituted heterocyclic group".

(Nail Cosmetic Material)

The nail cosmetic material of the present invention includes: diacetone acrylamide as a component 1; and at least one compound selected from the group consisting of isobornyl methacrylate, hydroxyethyl acrylamide, methacrylamide, dimethyl acrylamide, diethyl acrylamide, acryloyl morpholine, and butoxy diethylene glycol methacrylate, as a component 2.
The nail cosmetic material of the present invention can be suitably used not only as a composition for forming a gel nail or nail polish such as manicure or pedicure, but also as a nail protective agent such as a nail coat.
The nail cosmetic material of the present invention can suitably be used for any of a primer layer, a base layer, a color layer, and/or a top layer in an artificial nail. Among them, the nail cosmetic material of the present invention can suitably be used as a nail cosmetic material for a primer layer or a base layer and more suitably used as a nail cosmetic material for a base layer.
In addition, the nail cosmetic material of the present invention is preferably a photocurable composition.
The present inventors have conducted detailed studies. As a result, the present inventors have found that it is possible to obtain a nail cosmetic material having excellent adhesiveness and peelability by using both diacetone acrylamide and a specific monomer, and have completed the present invention.
Although the detailed mechanism in the present invention is unknown, since diacetone acrylamide is a monomer which is compatible with various high polarity monomers and low polarity monomers and has high adhesiveness to nails, it is estimated that diacetone acrylamide has concertedly excellent adhesiveness and peelability when being combined with a specific monomer.

Component 1: Diacetone Acrylamide

The nail cosmetic material of the present invention contains diacetone acrylamide as a component 1.
Diacetone acrylamide, denoted in English, is a compound described below.

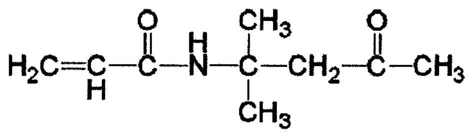

The content of diacetone acrylamide in the nail cosmetic material of the present invention is, with respect to 100 parts by mass of the nail cosmetic material, preferably 1 to 50 parts by mass and more preferably 5 to 30 parts by mass. In a case where diacetone acrylamide is within the above-described ranges, it is possible to achieve both high coatability and improved adhesion to nails.
Component 2: at least one compound selected from the group consisting of isobornyl methacrylate, hydroxyethyl acrylamide, methacrylamide, dimethyl acrylamide, diethyl acrylamide, acryloyl morpholine, and butoxy diethylene glycol methacrylate
The nail cosmetic material of the present invention contains at least one compound selected from the group consisting of isobornyl methacrylate, hydroxyethyl acrylamide, methacrylamide, dimethyl acrylamide, diethyl acrylamide, acryloyl morpholine, and butoxy diethylene glycol methacrylate, as a component 2. In a case of using both of the component 1 and the component 2, adhesiveness and peelability become excellent.
In addition, it is preferable that the nail cosmetic material of the present invention further contains at least two compounds selected from the group consisting of isobornyl methacrylate, hydroxyethyl acrylamide, methacrylamide, dimethyl acrylamide, diethyl acrylamide, acryloyl morpholine, and butoxy diethylene glycol methacrylate, as components 2. In the case of the embodiment, the adhesiveness and the peelability become more excellent.
In addition, among them, the component 2 preferably contains at least one compound selected from the group consisting of isobornyl methacrylate, hydroxyethyl acrylamide, methacrylamide, and dimethyl acrylamide, more preferably contains isobornyl methacrylate; and at least one compound selected from the group consisting of hydroxyethyl acrylamide, methacrylamide, and dimethyl acrylamide, and particularly preferably contains isobornyl methacrylate, hydroxyethyl acrylamide, and methacrylamide. In the case of the embodiment, the adhesiveness and the peelability become more excellent.
Among the monomers, isobornyl methacrylate is suitable from the viewpoint of improving film strength and water resistance of the nail cosmetic material, and it is estimated that hydroxyethyl acrylamide, methacrylamide, and dimethyl acrylamide has an effect of achieving both adhesiveness to nails and high film strength through hydrogen bond.

Thus, it is estimated that adhesiveness can be maximized using diacetone acrylamide which can dissolve both parties at the same time.

The total content of the components 2 in the nail cosmetic material of the present invention is, with respect to 100 parts by mass of the nail cosmetic material, preferably 1 to 80 parts by mass, more preferably 5 to 60 parts by mass, and still more preferably 5 to 30 parts by mass. In a case where the total content of the components 2 is within the above-described ranges, the adhesiveness becomes more excellent and the film strength of a cured film to be obtained becomes excellent.

In addition, the content ratio (mass ratio) of the component 1 to the components 2 in the nail cosmetic material of the present invention, that is, content of component 1:total content of components 2, is preferably 5:1 to 1:5, more preferably 4:1 to 1:4, and particularly preferably 3:1 to 1:3.

<Other Ethylenically Unsaturated Compounds>

The nail cosmetic material of the present invention may contain an ethylenically unsaturated compound other than the component 1 and the components 2.

The ethylenically unsaturated compound other than the component 1 and the components 2 may be a monofunctional ethylenically unsaturated compound or a polyfunctional ethylenically unsaturated compound, but is preferably a monofunctional ethylenically unsaturated compound.

In addition, the ethylenically unsaturated compound at this time the component 1 and the components 2 is preferably a compound having a molecular weight of less than 2,000, and more preferably a compound having a molecular weight of less than 1,000.

Preferred examples of the ethylenically unsaturated compound other than the component 1 and the components 2 include a (meth)acrylic acid amide compound, a (meth)acrylic acid ester compound, (meth)acrylic acid, a styrene compound, and N-vinyl amide compound other than the component 1 and the components 2.

Specific examples of the ethylenically unsaturated compound other than the component 1 and the components 2 include (meth)acrylic acid; monofunctional (meth)acrylic acid esters such as methyl (meth)acrylate, ethyl (meth)acrylate, 2-ethyl hexyl (meth)acrylate, 2-hydroxyethyl (meth)acrylate, 2-hydroxypropyl (meth)acrylate, 2-hydroxybutyl (meth)acrylate, isobornyl acrylate, and glycerin monomethacrylate; N-vinyl amides such as N-vinyl pyrrolidone and N-vinyl caprolactam; styrenes such as styrene, 4-acetoxystyrene, 4-carboxystyrene, and the like; polyfunctional (meth)acrylic acid esters such as ethylene glycol di(meth)acrylate, triethylene glycol di(meth)acrylate, tetraethylene glycol di(meth)acrylate, propylene glycol di(meth)acrylate, trimethylolpropane tri(meth)acrylate, pentaerythritol tri(meth)acrylate, pentaerythritol tetra(meth)acrylate, dipentaerythritol penta(meth)acrylate, dipentaerythritol hexa(meth)acrylate, 1,6-hexanediol di(meth)acrylate, cardo epoxy di(meth)acrylate, and neopentyl glycol di(meth)acrylate; urethane (meth)acrylate including diisocyanate- and hydroxyl group-containing (meth)acrylic acid derivatives and optionally including diols.

Among the above-described ethylenically unsaturated compounds, at least one compound selected from the group consisting of isobornyl acrylate, acrylic acid, methacrylic acid, N-vinyl pyrrolidone, N-vinyl caprolactam, 2-hydroxyethyl methacrylate, 2-hydroxypropyl methacrylate, 2-hydroxybutyl methacrylate, and glycerin monomethacrylate, and more preferably at least one compound selected from the group consisting of isobornyl acrylate, acrylic acid, 2-hydroxyethyl methacrylate, and 2-hydroxypropyl methacrylate.

The ethylenically unsaturated compound other than the component 1 and the components 2 may be used singly or in combination of two or more thereof.

The content of the ethylenically unsaturated compound other than the component 1 and the components 2 in the nail cosmetic material of the present invention is, with respect to 100 parts by mass of the nail cosmetic material, preferably 0 to 50 parts by mass, more preferably 5 to 40 parts by mass, and still more preferably 10 to 30 parts by mass. In a case where the content of the ethylenically unsaturated compound other than the component 1 and the components 2 is within the above-described ranges, adhesiveness and removability of a cured product become more excellent and production of the nail cosmetic material is facilitated.

<Photopolymerization Initiator>

The nail cosmetic material of the present invention preferably contains a photopolymerization initiator and more preferably contains a photoradical polymerization initiator.

Any well-known photopolymerization initiator can be suitably used as the photopolymerization initiator. Although specific examples of the photopolymerization initiator will be shown below, the present invention is not limited thereto.

Acetophenone compounds (for example, 1-hydroxycyclohexyl phenyl ketone, 2,2-dimethoxy-2-phenylacetophenone, 2-hydroxy-2-methyl propiophenone, 4'-isopropyl-2-hydroxy-2-methyl propiophenone, 2-methyl-1-[4-(methylthio)phenyl]-2-morpholino-1-propane, 2-benzyl-2-dimethylamino-1-(4-morpholinophenyl)-butan-1-one, 1-[4-(2-hydroxyethoxy)phenyl]-2-hydroxy-2-methyl-1-propan-1-one, 1-[4-(2-(2-hydroxyethoxy)ethoxy)phenyl]-2-hydroxy-2-methyl-1-propan-1-one, benzoin, benzoin methyl ether);

Benzophenone compounds (for example, 4,4'-bis(dimethylamino) benzophenone, or 3,3 -dimethyl-4-methoxybenzophenone);

Anthraquinone compounds (for example, anthraquinone, 2-methyl anthraquinone, 2-ethyl anthraquinone, or tert-butyl anthraquinone);

Thioxanthone compounds (for example, 2-chlorothioxanthone, diethyl thioxanthone, isopropyl thioxanthone, or diisopropyl thioxanthone);

Trihaloalkyl compounds (for example, 2,4,6-(trichloromethyl)triazine, 2,4-trichloromethyl-6-(4-methoxyphenyl)triazine, or tribromomethyl phenyl sulfone);

Lophine dimer compounds (for example, 2-(o-chlorophenyl)-4,5-diphenylimidazolyl dimer);

Acridine compounds (for example, 9-phenylacridine, 1,7-bis(9-acridinyl)heptane, 1,5-bis(9-acridinyl)pentane, or 1,3-bis(9-acridinyl)propane);

Phosphine oxide compounds (for example, trimethylbenzoyl diphenylphosphine oxide);

Metallocene compounds (for example, biscyclopentadienyl bis(difluoropyrrylphenyl)titanium); and

Onium salt compounds (for example, bis(4-t-butylphenyl)iodonium tosylate or triphenyl sulfonium tosylate).

The photopolymerization initiator can be appropriately selected according to the wavelength of light emitted by a light source used for exposure. However, at least one compound selected from the group consisting of an acetophenone compound, a benzophenone compound, a thioxanthone compound, a phosphine oxide compound, a metallocene compound, and a lophine dimer compound is preferably used, and at least one compound selected from the group consisting of acetophenone compound, a benzophenone compound, a thioxanthone compound, and a phosphine oxide compound is more preferably used.

In addition, the photopolymerization initiator can be appropriately selected according to the composition of the nail cosmetic material. Specifically, a photopolymerization initiator to be used is preferably selected so as to be dissolved in the component 1, the components 2, the ethylenically unsaturated compound other than the component 1 and the components 2, and/or a solvent to be described below.

The photopolymerization initiator may be used singly or in combination of two or more thereof.

The content of the photopolymerization initiator in the nail cosmetic material of the present invention is, with respect to 100 parts by mass of the nail cosmetic material, preferably 0.1 to 20 parts by mass, more preferably 0.5 to 15 parts by mass, and particularly preferably 1 to 10 parts by mass. In a case where the content of the photopolymerization initiator is within the above-described ranges, the adhesiveness of a cured product becomes more excellent.

<Polymer and/or Oligomer>

The nail cosmetic material of the present invention preferably contains a polymer and/or an oligomer. In a case where the nail cosmetic material contains a polymer and/or an oligomer, the adhesiveness becomes more excellent and the film strength of a cured film becomes excellent.

The weight-average molecular weight of the polymer and/or the oligomer used in the present invention is preferably 2,000 to 300,000, more preferably 3,000 to 200,000, still more preferably 4,000 to 150,000, and particularly preferably 5,000 to 100,000. In a case where the weight-average molecular weight of the polymer and/or the oligomer is within the above-described ranges, adhesiveness and temporal stability of an obtained cured product become excellent.

In the present invention, a compound having a weight-average molecular weight of greater than or equal to 2,000 and less than 5,000 is called an oligomer and a compound having a weight-average molecular weight of greater than or equal to 5,000 is called a polymer.

A method for measuring the weight-average molecular weight of the polymer and the oligomer in the present invention is performed through gel permeation chromatography based on a polystyrene standard or a polyethylene oxide standard.

The structure of the polymer and/or the oligomer that can be used in the present invention is not particularly limited, and an arbitrary structure may be used. Examples thereof include a chain structure, a branched structure, a star structure, a crosslinking structure, and a network structure.

The types of polymers and/or oligomers are not particularly limited, and any well-known types of polymers (poly(meth)acrylic acid ester, poly(meth)acrylic acid amide, polyurethane, polyester, polyether, polyurea, polycarbonate, polyamide, polystyrene, polyalkylene, and polyvinyl) can be used.

The polymer and/or the oligomer preferably have a radically polymerizable ethylenically unsaturated group, and more preferably have a (meth)acrylic group.

The polymer and/or the oligomer are preferably a polymer and/or an oligomer having a structure represented by Formula C-1, and are particularly preferably a polymer and/or an oligomer having the structure represented by Formula C-1 in a polymer main chain. In the case of the embodiment, the adhesiveness becomes particularly excellent.

The polymer and/or the oligomer are more preferably a polymer and/or an oligomer having a urethane bond,

and are particularly preferably a polymer and/or an oligomer having a urethane bond in a polymer main chain. In the case of the embodiment, the adhesiveness becomes particularly excellent.

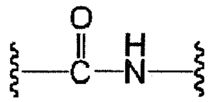 (C-1)

The wavy line portions each independently represent a bonding position with other structures.
Specific examples of the polymer and/or the oligomer used in the present invention are shown below, but the present invention is not limited thereto.

[Table 1]

| Number | Unit-1 | | Unit-2 | | Unit-3 | | Unit-4 | | Unit-5 | | Mw |
|---|---|---|---|---|---|---|---|---|---|---|---|
| P-16 | TDI | 50 | Diol-9 | 30 | Diol-5 | 18 | HEMA | 2 | - | - | 20,000 |
| P-17 | TDI | 50 | Diol-1 | 30 | Diol-2 | 18 | HPMA | 2 | - | - | 20,000 |
| P-18 | MDI | 40 | HDI | 10 | Diol-6 | 18 | Diol-10 | 30 | Methanol | 2 | 22,000 |
| P-19 | ADPA | 50 | Diamine-2 | 48 | n-butylamine | 2 | - | - | - | - | 30,000 |
| P-20 | MMA | 50 | BMA | 50 | - | - | - | - | - | - | 35,000 |
| P-21 | ADPA | 50 | Diol-8 | 48 | Methanol | 2 | - | - | - | - | 15,000 |
| P-22 | Nitrocellulose | | | | | | | | | | |

The numerical values denoted in the columns of each unit in Table 1 represent molar ratios of monomer units derived from compounds forming each unit.
The details of the compounds forming each unit described in Table 1 are shown below.

TDI: tolylene diisocyanate
Diol-9: 2,2-bis(4-hydroxycyclohexyl)propane
Diol-5: polytetramethylene oxide (polytetramethylene ether glycol, Mw:2,000)
HEMA: 2-hydroxyethyl methacrylate
Diol-1: 3-dimethylamino-1,2-propanediol
Diol-2: polycarbonate diol composed of 1,5-penthanediol and 1,6-hexanediol (Mw: 2,000)
HPMA: 2-hydroxypropyl methacrylate
MDI: bis(4-isocyanatophenyl)methane
HDI: hexamethylene diisocyanate
Diol-6: polypropylene glycol (Mw: 1,000)
Diol-10: glycerin monomethacrylate
MMA: methyl methacrylate
BMA: n-butyl methacrylate
ADPA: adipic acid dichloride
Diol-8: 1,4-butanediol
Nitrocellulose: DHX40-70 (manufactured by Inabata & Co., Ltd)
Diamine-2: polypropylene glycol diamine (Mw: 2,000)

The polymer and/or the oligomer used in the present invention are preferably a polymer and/or an oligomer having an amino group.
Any of primary, secondary, and tertiary amino groups can be used as the amino groups. However, a tertiary amino group is preferable from the viewpoints of easiness of production of a polymer and/or an oligomer, adhesiveness and removability of an obtained cured film, and temporal stability.
The introduction position of an amino group, particularly a tertiary amino group, in the polymer and/or the oligomer may be any of the side chain, the inside of a main chain, and a terminal of a main chain, and may be two or more positions.
A group represented by Formula C-2 or Formula C-3 is preferable as the tertiary amino group.

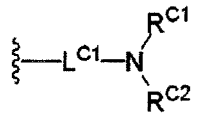

(C-2)

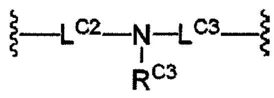

(C-3)

In Formula C-2, $R^{C1}$ and $R^{C2}$ each independently represent a hydrogen atom or an alkyl group having 1 to 10 carbon atoms, $L^{C1}$ represents a divalent linking group; at least two selected from the group consisting of $R^{C1}$, $R^{C2}$, and $L^{C1}$ may be linked to each other to form a ring; and the wavy line portions represent linking positions with other structures.

$R^{C3}$ in Formula C-3 represents a hydrogen atom or an alkyl group having 1 to 10 carbon atoms; $L^{C2}$ and $L^{C3}$ each independently represent a divalent linking group; at least two selected from the group consisting of $R^{C3}$, $L^{C2}$, and $L^{C3}$ may be linked to each other to form a ring; and the wavy line portions each independently represent linking positions with other structures.

$R^{C1}$ and $R^{C2}$ in Formula C-2 is preferably an alkyl group having 1 to 10 carbon atoms, more preferably an alkyl group having 1 to 6 carbon atoms, still more preferably an alkyl group having 1 to 4 carbon atoms, and particularly preferably an alkyl group having 1 or 2 carbon atoms from the viewpoint of a balance between solubility in an aqueous acid solution and water resistance.

Examples of $L^{C1}$ in Formula C-2 include a single bond, an alkylene group having 1 to 20 carbon atoms (which may have a substituent or in which some carbon atoms may be substituted with hetero atoms), and an arylene group having 1 to 20 carbon atoms (which may have a substituent or in which some carbon atoms may be substituted with hetero atoms). $L^{C1}$ in Formula C-2 is preferably a single bond, an alkylene group having 1 to 20 carbon atoms, an oxyalkylene group having 2 to 20 carbon atoms, or a polyoxyalkylene group having 2 to 20 carbon atoms, more preferably an alkylene group having 1 to 20 carbon atoms or a polyoxyalkylene group having 2 to 20 carbon atoms, particularly preferably an alkylene group having 1 to 20 carbon atoms, and most preferably an alkylene group having 1 to 10 carbon atoms.

In Formula C-2, at least two selected from the group consisting of $R^{C1}$, $R^{C2}$, and $L^{C1}$ may be linked to each other to form a ring.

$R^{C3}$ in Formula C-3 is preferably an alkyl group having 1 to 10 carbon atoms, more preferably an alkyl group having 1 to 6 carbon atoms, and still more preferably an alkyl group having 1 to 4 carbon atoms from the viewpoint of a balance between solubility in an aqueous acid solution and water resistance.

Examples of $L^{C2}$ and $L^{C3}$ in Formula C-3 each independently include a single bond, an alkylene group having 1 to 20 carbon atoms (which may have a substituent or in which some carbon atoms may be substituted with hetero atoms), and an arylene group having 1 to 20 carbon atoms (which may have a substituent or in which some carbon atoms may be substituted with hetero atoms). $L^{C2}$ and $L^{C3}$ in Formula C-3 are preferably a single bond, an alkylene group having 1 to 20 carbon atoms, an oxyalkylene group having 2 to 20 carbon atoms, or a polyoxyalkylene group having 2 to 20 carbon atoms, more preferably an alkylene group having 1 to 20 carbon atoms or a polyoxyalkylene group having 2 to 20 carbon atoms, particularly preferably an alkylene group having 1 to 20 carbon atoms, and most preferably an alkylene group having 1 to 10 carbon atoms.

In Formula C-3, at least two selected from the group consisting of $R^{C3}$, $L^{C2}$, and $L^{C3}$ may be linked to each other to form a ring.

The amine value of the polymer and/or the oligomer used in the present invention is preferably 0.1 to 10 mmol/g, more preferably 0.25 to 9 mmol/g, and still more preferably 0.5 to 8 mmol/g. In a case where the amine value thereof is within the above-described ranges, the adhesiveness and the removability become more excellent.

As a method for measuring the amine value, it is possible to obtain the amine value such that, for example, a sample is weighed in a beaker, acetic acid is added to the sample, the mixture is stirred and dissolved, the measurement temperature is adjusted to be 25°C, and then, titration is performed with a titration device using a 0.1 N perchloric acid acetic acid solution as a titration reagent. The amine value is a value obtained by representing the amount of the perchloric acid consumed during the titration in terms of the number of moles per 1 g of the sample (solid content).

Specific examples of the polymer and/or the oligomer having an amino group used in the present invention will be shown below, but the present invention is not limited thereto.

[Table 2]

| Number | Unit-1 | | Unit-2 | | Unit-3 | | Unit-4 | | Unit-5 | | Mw |
|---|---|---|---|---|---|---|---|---|---|---|---|
| P-1 | IPDI | 50 | Diol-1 | 40 | Diol-2 | 8 | Methanol | 2 | - | - | 35,000 |
| P-2 | IPDI | 50 | Diol-3 | 40 | Diol-2 | 8 | Methanol | 2 | - | - | 34,000 |

(continued)

| Number | Unit-1 | | Unit-2 | | Unit-3 | | Unit-4 | | Unit-5 | | Mw |
|---|---|---|---|---|---|---|---|---|---|---|---|
| P-3 | IPDI | 50 | Diamine-1 | 39 | Diamine-2 | 9 | Morpholine | 2 | - | - | 13,000 |
| P-4 | ADPA | 50 | Diamine-1 | 39 | Diamine-2 | 9 | Morpholine | 2 | - | - | 22,000 |
| P-5 | ADPA | 50 | Diol-1 | 20 | Diol-4 | 28 | IPA | 2 | - | - | 35,000 |
| P-6 | DMAPAm | 50 | CHAm | 50 | - | - | - | - | - | - | 40,000 |
| P-7 | DMAEM | 50 | BMA | 50 | - | - | - | - | - | - | 45,000 |
| P-8 | IPDI | 50 | Diol-1 | 40 | Diol-2 | 8 | HEMA | 2 | - | - | 36,000 |
| P-9 | TDI | 50 | Diol-1 | 30 | Diol-5 | 18 | HEMA | 2 | - | - | 25,000 |
| P-10 | HDI | 50 | Diol-3 | 30 | Diol-5 | 18 | HEMA | 2 | - | - | 31,000 |
| P-11 | MDI | 50 | Diol-1 | 48 | Ethanol | 2 | - | - | - | - | 23,000 |
| P-12 | TDI | 50 | Diamine-1 | 39 | Diamine-2 | 9 | Piperidine | 2 | - | - | 10,000 |
| P-13 | ADPA | 50 | Diamine-2 | 48 | DMPA | 2 | - | - | - | - | 8,000 |
| P-14 | IPDI | 50 | Diamine-2 | 48 | DMPA | 2 | - | - | - | - | 9,000 |
| P-15 | MDI | 40 | HDI | 10 | Diol-6 | 10 | Diol-7 | 25 | Diol-8 | 15 | 73,000 |

The numerical values denoted in columns of each unit in Table 2 represent molar ratios of monomer units derived from compounds forming each unit.

The details of the compounds forming each unit described in Table 2 are shown below.

IPDI: isophorone diisocyanate
TDI: tolylene diisocyanate
HDI: hexamethylene diisocyanate
MDI: bis(4-isocyanatophenyl)methane
Diol-1: 3-dimethylamino-1,2-propanediol
Diol-2: polycarbonate diol composed of 1,5-penthanediol and 1,6-hexanediol (Mw: 2,000)
Diol-3: N-butyl diethanolamine
Diol-4: 1,5-penthanediol
Diol-5: polytetramethylene oxide (polytetramethylene ether glycol, Mw:2,000)
Diol-6: polypropylene glycol (Mw: 1,000)
Diol-7: 3-diethylamino-1,2-propanediol
Diol-8: 1,4-butanediol
Diamine-1: 3,3-diamine-N-methyl dipropylamine
Diamine-2: polypropylene glycol diamine (Mw: 2,000)
ADPA: adipic acid dichloride
DMAPAm: 3-dimethylaminopropyl acrylamide
CHAm: N-cyclohexyl acrylamide
DMAEM: 2-dimethylaminoethyl methacrylate
BMA: n-butyl methacrylate
HEMA: 2-hydroxyethyl methacrylate
IPA: 2-hydroxypropane
DMPA: 3-dimethylaminopropylamine

The polymer and/or the oligomer used in the present invention can be produced through a well-known method (for example, radical polymerization or polycondensation).

The polymer and/or the oligomer may be used singly or in combination of two or more thereof.

The content of the polymer and/or the oligomer in the nail cosmetic material used in the present invention is, with respect to the total mass of the nail cosmetic material, preferably greater than 0 mass% and less than or equal to 90 mass%, more preferably 5 to 80 mass%, and still more preferably 10 to 60 mass%. In a case where the content of the polymer and/or the oligomer is within the above-described ranges, the adhesiveness and the removability of a cured product become more excellent.

A water-soluble polymer and/or an oligomer can also be suitably used as the polymer and/or the oligomer used in the present invention.

The water-soluble polymer and/or the oligomer is preferably a polymer and/or an oligomer that dissolves in 100 g of distilled water in an amount of greater than or equal to 0.1 g, more preferably a polymer and/or an oligomer that dissolves in 100 g of distilled water in an amount of greater than or equal to 0.2 g, and particularly preferably a polymer and/or an oligomer that dissolves in 100 g of distilled water in an amount of greater than or equal to 0.5 g. In the case where the polymer and/or the oligomer have water solubility within the above-described ranges, production of a nail cosmetic material using water becomes facilitated.

A polymer and/or an oligomer having a functional group selected from the group consisting of a carboxylic acid (salt) group, a sulfonic acid (salt) group, a phosphoric acid (salt) group, a phosphonic acid (salt) group, a quaternary ammonium salt group, a hydroxyl Group, a carboxylic acid amide group, and a polyethylene glycol chain are preferable as the water-soluble polymer and/or the oligomer used in the present invention.

As counter cations of a carboxylic acid (salt) group, a sulfonic acid (salt) group, a phosphoric acid (salt) group, and a phosphonic acid (salt) group, alkali metal cations such as sodium and potassium, alkaline earth metal cations such as calcium and magnesium, an ammonium cation, and a phosphonium cation are preferable, and alkali metal cations such as sodium and potassium are particularly preferable.

A methyl group or an ethyl group is preferable as an alkyl group of an ammonium group of a quaternary ammonium salt group. In addition, as counter anions, halide ions such as a chloride ion and a bromide ion, a sulfate anion, a nitrate anion, a phosphate anion, a sulfonate anion, a carboxylate anion, and a carbonate anion are preferable, and a halide ion, a sulfonate anion, and a carboxylate anion are particularly preferable.

As a substituent on nitrogen of a carboxylic acid amide group, an alkyl group having less than or equal to 8 carbon atoms is preferable, and an alkyl group having less than or equal to 6 carbon atoms is particularly preferable.

The linking chain length of polyethylene glycol chains is preferably greater than or equal to 2 and particularly preferably greater than or equal to 4.

Specific examples of the water-soluble polymer and/or the oligomer used in the present invention will be shown below, but the present invention is not limited thereto.

[Table 3]

| Number | Unit-1 | | Unit-2 | | Unit-3 | | Unit-4 | | Unit-5 | | Mw |
|---|---|---|---|---|---|---|---|---|---|---|---|
| P-23 | MDI | 50 | Diol-6 | 5 | Diol-11 | 25 | Diol-10 | 18 | Methanol | 2 | 100,000 |
| P-24 | TDI | 50 | Diol-12 | 18 | Diol-9 | 30 | HEMA | 2 | - | - | 20,000 |
| P-25 | Polymer represented by Formula C' | | | | | | | | | | 20,000 |

The numerical values denoted in columns of each unit in Table 3 represent molar ratios of monomer units derived from compounds forming each unit.

The details of the compounds forming each unit described in Table 3 are shown below.

MDI: bis(4-isocyanatophenyl)methane
Diol-6: polypropylene glycol (Mw: 1,000)
Diol-11: sodium 2,2-bis(hydroxymethyl) propionate
Diol-10: glycerin monomethacrylate
TDI: tolylene diisocyanate
Diol-12: polyethylene glycol (Mw: 2,000)
Diol-9: 2,2-bis(4-hydroxycyclomethyl)propane

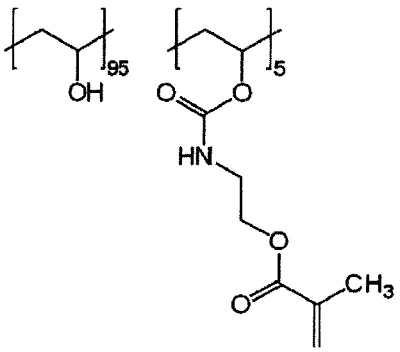

(C')

The figures on the lower right sides of the parentheses in the polymer represented by Formula C' represent molar ratios.

The polymer and/or the oligomer used in the present invention can be produced through a well-known method (for example, radical polymerization or polycondensation).

The polymer and/or the oligomer may be used singly or in combination of two or more thereof.

The content of the polymer and/or the oligomer in the nail cosmetic material used in the present invention is, with respect to 100 parts by mass of the nail cosmetic material, preferably greater than 0 parts by mass and less than or equal to 90 parts by mass, more preferably 5 to 80 parts by mass, and still more preferably 10 to 60 parts by mass. In a case where the content of the polymer and/or the oligomer is within the above-described ranges, the adhesiveness and the removability of a cured product become more excellent.

<Solvent>

The nail cosmetic material of the present invention may contain a solvent in order to dissolve each component or to adjust easiness of applying the nail cosmetic material.

As the solvent, a solvent having a boiling point of 50°C to 150°C at 1 atmosphere (1013.25 hPa) is preferable, and a solvent having a boiling point of 60°C to 140°C at 1 atmosphere is more preferable. In a case where a solvent having a boiling point within the above-described ranges, concentration fluctuation of the nail cosmetic material can be suppressed and drying after coating is favorably performed.

Examples of the solvent include: water; alcohols such as methanol, ethanol, isopropyl alcohol (2-propanol), 1-propanol, 1-butanol, 2-butanol, t-butyl alcohol, 2-methyl-1-propanol, and 1-methoxy-2-propyl alcohol; hydrocarbons such as hexane, heptane, toluene, and xylene; ethers such as diethyl ether, diisopropyl ether, tetrahydrofuran, dimethoxyethane, and diphenyl ether; esters such as methyl acetate, ethyl acetate, butyl acetate, and γ-butyrolactone; ketones such as acetone, methyl ethyl ketone, and cyclohexanone; amides such as N,N-dimethylformamide, N,N-dimethylacetamide, N-methylpyrrolidone, and N-ethylpyrrolidone; carbonates such as dimethyl carbonate, diethyl carbonate, and propylene carbonate; ureas such as tetramethyl urea and 1,3-dimethyl-2-imidazolidinone; halogenated hydrocarbons such as chloroform, dichloromethane, and chlorobenzene; and well-known solvents such as triethylamine, acetic acid, acetonitrile, nitromethane, and dimethyl sulfoxide.

Among these solvents, water, methanol, ethanol, isopropyl alcohol, 1-propanol, 1-butanol, 2-butanol, t-butyl alcohol, 2-methyl-1-propanol, and 1-methoxy-2-propyl alcohol, acetic acid, and/or acetonitrile are preferable, and water, ethanol, isopropyl alcohol, and/or acetic acid are more preferable. By using the solvents, it is possible to reduce the viscosity and to improve the coatability.

The solvent may be contained singly or in combination of two or more thereof.

In a case where the nail cosmetic material of the present invention contains the solvents, the content of the solvent in the nail cosmetic material of the present invention is, with respect to 100 parts by mass of the nail cosmetic material, preferably greater than 0 part by mass and less than or equal to 80 parts by mass, more preferably 5 to 70 parts by mass, and still more preferably 10 to 60 parts by mass. In a case where the content of the solvent is within the above-described ranges, the coatability and the drying properties of the nail cosmetic material become favorable.

In addition, regarding the mixing ratio of diacetone acrylamide to the solvent, diacetone acrylamide is, with respect to 100 parts by mass of the solvent, preferably 5 to 200 parts by mass, more preferably 7 to 150 parts by mass, and still more preferably 10 to 100 parts by mass. In a case where the mixing ratio thereof is within the above-described ranges, the production of the nail cosmetic material becomes facilitated.

<Other Components>

In addition to the above-described components, any well-known compounds can be added to the nail cosmetic material of the present invention as necessary. Specific examples thereof include additives such as a sensitizer, a polymerization inhibitor, a pigment, a dye, a perfume, an ultraviolet (UV) absorbent, an antioxidant, a filler, various elastomers, a plasticizer, a thickener, a thixotropy imparting agent, a silane coupling agent, a titanate coupling agent, a chelating agent, a flame retardant, and a surfactant.

<Method for Applying Nail Cosmetic Material>

Examples of the method for applying a nail cosmetic material using the nail cosmetic material of the present invention preferably include a method for coating an object such as a nail, a nail on which other nail cosmetic materials are applied, or an artificial nail with the nail cosmetic material of the present invention to form a cured product while irradiating the nail cosmetic material with light.

The method for coating an object with the nail cosmetic material of the present invention is not particularly limited, and may be performed through well-known methods. Examples thereof include a method for coating the nail cosmetic material using a paint brush, an ink brush, or the like, spray coating, inkjet coating, bar coater coating, rotary coating, curtain coating, dip coating, air knife coating, blade coating, and roll coating.

The coating thickness of the nail cosmetic material of the present invention on an object varies in accordance with its use. The film thickness after curing is preferably 1 nm to 1 mm, more preferably 10 nm to 0.5 mm, and still more preferably 100 nm to 0.25 mm. In a case where the film thickness is within the above-described ranges, more favorable adhesiveness and removability are achieved.

The method for coating an object with the nail cosmetic material of the present invention and irradiating the nail cosmetic material with light is not particularly limited. Any well-known light irradiation method (for example, whole surface exposure or scanning exposure) can be used in which a well-known light source (for example, solar light, a high pressure mercury lamp, a fluorescent lamp, a UV lamp, a light emitting diode (LED) lamp, or an LED laser) is used. The light irradiation time is not particularly limited as long as the nail cosmetic material of the present invention is cured.

As the method for forming a cured product of the nail cosmetic material on an object, the above-described method can be repeatedly performed.

As a method for removing a cured film obtained by curing the nail cosmetic material of the present invention, the nail cosmetic material can be removed through well-known methods. Specific examples thereof include a polishing method using a file or the like; a scraping method using a sculpture knife, a knife, or the like; a method for covering a cured film with cotton moistened with acetone and allowing the cured film to stand for a few minutes to make the cured film brittle, and then, peeling or crushing the cured film with a bar or the like; a method for immersing a cured film in an aqueous solution having a specific pH for a few minutes to make the cured film brittle, and then, peeling the cured film with tweezers or the like or pushing and scraping the cured film with a bar; and a method in which these methods are combined.

Particularly, preferred examples of the method for removing a cured film obtained by curing the nail cosmetic material of the present invention from a nail include method for covering a cured film with cotton moistened with acetone and allowing the cured film to stand for a few minutes to make the cured film brittle, and then, peeling or crushing the cured film with a bar or the like; a method for immersing a cured film in an aqueous solution having a specific pH for a few minutes to make the cured film brittle, and then, peeling the cured film with tweezers or the like or pushing and scraping the cured film with a bar; and a method in which these methods are combined. More preferred example of the method for removing a cured film obtained by curing the nail cosmetic material of the present invention from a nail include a method for immersing a cured film in an aqueous solution having a specific pH for a few minutes to make the cured film brittle, and then, peeling the cured film with tweezers or the like or pushing and scraping the cured film with a bar.

<Nail Art Kit>

The nail art kit of the present invention includes the nail cosmetic material of the present invention and one or more tools for applying the nail cosmetic material on a nail.

A preferred embodiment of the nail cosmetic material of the present invention in the nail art kit of the present invention is as described above.

Specific examples of the tools for applying a nail cosmetic material on a nail include a nail cosmetic material other than the nail cosmetic material of the present invention for color layer, a top layer, or the like; a nail file such as a file; an ink brush such as a flat brush, or a paint brush, for applying a nail cosmetic material; a device of exposing UV light or the like; a wiping or cleaning liquid; a wipe for wiping; a nail brush; a dust brush; a nail form used for repair of a nail, decorative stones made of acryl, glass, metal, natural stone, or the like; nail seal; decorative powder such as glitter

or hologram; a cutter; a spatula; a stick; tweezers; and a separator for separating the distances between fingers in order to prevent contact between nails. However, the present invention is not limited thereto.

[Examples]

Hereinafter, the present invention will be more specifically described using examples, but the present invention is not limited to embodiments in these examples. Unless otherwise specified, "parts" and "%" are on a mass basis.
In addition, polymers P-8, P-9, P-16, P-24, and P-25 used in the examples are respectively the same polymers as those described above.

(Examples 1 to 24 and Comparative Examples 1 to 8)

1. Preparation of Nail Cosmetic Material

Monomers described in Table 4 were placed in a container, provided with a stirring device, in amount described in Table 4. Then, the monomers were heated to 55°C using a heating and stirring device, and were mixed with each other until the monomers became homogeneous. Next, a urethane methacrylate polymer (P-8) and a photopolymerization initiator were weighed in a separate container, and a necessary amount of the monomer solution was weighed and mixed therewith to obtain a nail cosmetic material of Example 1.

<Composition of Nail Cosmetic Material of Example 1>

- Diacetone acrylamide (DAAA manufactured by Nippon Kasei Chemicals Co., Ltd.): 30 parts by mass
- Isobornyl methacrylate (IBXMA manufactured by KYOEISHA CHEMICAL Co., LTD): 10 parts by mass
- 2-Hydroxyethyl methacrylate (HEMA manufactured by KYOEISHA CHEMICAL Co., LTD): 15 parts by mass
- Acrylic acid: 5 parts by mass
- Urethane methacrylate polymer (P-8): 40 parts by mass
- Diphenyl(2,4,6-trimethylbenzoyl)phosphine oxide (TPO, photopolymerization initiator, LUCIRIN TPO manufactured by BASF): 4.5 parts by mass

Nail cosmetic materials of Examples 2 to 24 and Comparative Examples 1 to 8 were obtained through the same method as that in the preparation of the nail cosmetic material of Example 1 except that the types and the addition amounts of monomers to be used were changed as shown in Tables 4 to 7.

<Evaluation of Coatability>

[Production of Sample]

Nails of the hand were coated with the obtained nail cosmetic materials using a paint brush, and the coatability (stringiness or self-leveling properties) was evaluated.

-Evaluation Criteria-

3: There is no stringiness, and smoothing of the coated surface proceeds sufficiently after 10 seconds.
2: There is slight stringiness, and the coated surface has not been smoothed sufficiently even after 10 seconds.
1: There is stringiness, and the coated surface is not smoothed even after 10 seconds.

<Evaluation of Film Strength>

[Production of Sample]

A polyethylene terephthalate (PET) film was coated with each of the obtained nail cosmetic material in a film thickness of 100 μm, a length of 4 cm, and a width of 4 cm, the coating was irradiated with an ultraviolet lamp (36 W) for 2 hours to produce each sample.

[Gelation Rate]

The sample obtained from each of the nail cosmetic materials was immersed in 30 g of tetrahydrofuran (THF) for 24 hours. Accordingly, components other than gel components in the sample were dissolved in a solvent. The immersed sample was taken out and dried, and the mass of the sample was measured. Then, the gelation rate in the sample was calculated from the following Formula.

"Gelation rate" = (mass of sample after immersion) / (mass of sample before immersion) x 100 (%)

-Evaluation Criteria-

3: Gelation rate is greater than or equal to 80%
2: Gelation rate is greater than 70% and less than 80%
1: Gelation rate is less than or equal to 70%

<Evaluation of Adhesiveness and Peelability>

1. Formation of Base Coat Layer (Base Layer)

After nails of the hand were coated with each of the obtained nail cosmetic materials using a paint brush, the nail cosmetic materials were photocured (exposed for 60 seconds) using a gel nail exclusive UV device (36 W) to form base coat layers. The thickness of each formed base coat layer was about 100 $\mu$m.

2. Formation of Gel Nail Layer

Next, each obtained base coat layer was coated with commercially available CALGEL #CG-03 FRESH PINK (manufactured by MOGA BROOK Co., ltd.) using a paint brush, and then, irradiation was performed for 1 minute using an ultraviolet lamp (36 W) which is a gel nail exclusive UV device. Thereafter, the base coat layer was coated with commercially available TOP GEL (manufactured by MOGA BROOK Co., ltd.) as a top layer using a paint brush, and irradiation was similarly performed for 2 minutes using the ultraviolet lamp (36W). Each gel nail layer formed was visually observed, and as a result, the gel nail layer was completely cured. The thickness of each film in which the color layer and the top layer were combined was about 250 $\mu$m.

3. Evaluation

(1) Evaluation of Adhesiveness

(1)-1 External Force Resistance

The adhesiveness of each obtained nail/base coat layer/gel nail layer laminate was evaluated from the viewpoint of external force resistance. That is, a distal portion of each nail including a base coat layer and a gel nail layer was rubbed with the top of a commercially available rubber sheet 30 times, and then, the change in the distal portion was evaluated according to the following criteria. The obtained results are shown in Tables 4 to 7. In all cases, peeling between each base coat layer and each gel nail layer was not checked.

-Evaluation Criteria-

4: No change in appearance was checked.
3: Slight peeling was checked between a nail and a base coat layer.
2: Partial peeling was checked between a nail and a base coat layer, and whitening was checked in a part of the appearance.
1: Significant peeling was checked between a nail and a base coat layer, and whitening was checked on the whole

appearance.

(1)-2 Warm Water Resistance

In addition, the adhesiveness of each obtained nail/base coat layer/gel nail layer laminate was evaluated from the viewpoint of warm water resistance. That is, an operation of washing the hand, in a state where a base coat layer and a gel nail layer were formed on nails, was washed with warm water at about 40°C without using soap, and wiping the hand with towel material after the washing was finished was performed 30 times per day. The change in appearance after continuously performing this operation for 7 days was evaluated according to the following criteria. The obtained results are shown in Tables 4 to 7. In all cases, peeling between each base coat layer and each gel nail layer was not checked.

-Evaluation Criteria-

4: No change in appearance was checked.
3: Slight peeling was checked between a nail and a base coat layer.
2: Partial peeling was checked between a nail and a base coat layer, and whitening was checked in a part of the appearance.
1: Significant peeling was checked between a nail and a base coat layer, and whitening was checked on the whole appearance.

(2) Evaluation of Peelability

The peelability of each obtained nail/base coat layer/gel nail layer laminate was evaluated. That is, cloth containing acetone was wound on each nail on which a base coat layer and a gel nail layer were formed, and the nail was allowed to stand for 15 minutes. Then, the laminate was lightly scrubbed with a commercially available orange stick to check whether or not it is possible to peel the laminate. In a case where it was impossible to peel the laminate in one cycle of this operation, the same cycle was repeatedly performed, and the peelability was evaluated using the number of cycles, required for the laminate to be peeled, according to the following criteria. The obtained results are shown in Tables 4 to 7. In all cases, peeling between each base coat layer and each gel nail layer was not checked.

-Evaluation Criteria-

4: A laminate of a base coat layer and a gel nail layer was peeled from a nail in one cycle.
3: A laminate of a base coat layer and a gel nail layer was peeled from a nail in two cycles.
2: A laminate of a base coat layer and a gel nail layer was peeled from a nail in three cycles.
1: A laminate of a base coat layer and a gel nail layer was not peeled from a nail even in three cycles.

[Table 4]

| | Monomer | | | | Amount of monomers (parts by mass) | Polymer | Total (parts by mass) | Photopolymerization initiator | Coatability | Film strength | Adhesiveness | | Peelability |
|---|---|---|---|---|---|---|---|---|---|---|---|---|---|
| | DAAA | IBXMA | HEMA | Acrylic acid | | P-8 | | TPO | | | External force resistance | Warm water resistance | |
| Example 1 | 30 | 10 | 15 | 5 | 60 | 40 | 100 | 4.5 | 3 | 3 | 3 | 3 | 2 |
| Example 2 | 20 | 20 | 15 | 5 | 60 | 40 | 100 | 4.5 | 3 | 3 | 3 | 3 | 3 |
| Example 3 | 15 | 25 | 15 | 5 | 60 | 40 | 100 | 4.5 | 3 | 3 | 2 | 3 | 3 |
| Comparative Example 1 | 40 | - | 15 | 5 | 60 | 40 | 100 | 4.5 | 3 | 2 | 3 | 1 | 2 |
| Comparative Example 2 | - | 40 | 15 | 5 | 60 | 40 | 100 | 4.5 | 3 | 2 | 2 | 3 | 1 |

[Table 5]

| | Monomer | | | | | | | | | Amount of monomers (parts by mass) | Polymer | Total (parts by mass) | Photopolymerization initiator | Coatability | Film strength | Adhesiveness | | Peelability |
|---|---|---|---|---|---|---|---|---|---|---|---|---|---|---|---|---|---|---|
| | DAAA | HEAA | MAm | DMAA | DEAA | ACMO | Light ester BC | HPMA | IBXA | | P-9 | | TPO | | | External force resistance | Warm water resistance | |
| Example 4 | 20 | 15 | - | - | - | - | - | 10 | 10 | 55 | 45 | 100 | 4.0 | 3 | 3 | 4 | 2 | 3 |
| Example 5 | 20 | - | 15 | - | - | - | - | 10 | 10 | 55 | 45 | 100 | 4.0 | 3 | 3 | 4 | 3 | 2 |
| Example 6 | 20 | - | - | 15 | - | - | - | 10 | 10 | 55 | 45 | 100 | 4.0 | 3 | 3 | 3 | 3 | 3 |
| Example 7 | 20 | - | - | - | 15 | - | - | 10 | 10 | 55 | 45 | 100 | 4.0 | 3 | 3 | 3 | 3 | 2 |
| Example 8 | 20 | - | - | - | - | 15 | - | 10 | 10 | 55 | 45 | 100 | 4.0 | 3 | 3 | 4 | 3 | 3 |
| Example 9 | 20 | - | - | - | - | - | 15 | 10 | 10 | 55 | 45 | 100 | 4.0 | 3 | 3 | 3 | 3 | 3 |
| Comparative Example 3 | - | 15 | - | - | - | - | - | 10 | 30 | 55 | 45 | 100 | 4.0 | 1 | - | - | - | - |
| Comparative Example 4 | - | - | 15 | - | - | - | - | 10 | 30 | 55 | 45 | 100 | 4.0 | 2 | 3 | 2 | 1 | 2 |
| Comparative Example 5 | - | - | - | - | - | 15 | - | 10 | 30 | 55 | 45 | 100 | 4.0 | 3 | 2 | 2 | 2 | 1 |

[0067] [Table 6]

| | Monomer | | | | | | | | | | | | Amount of monomers (parts by mass) | Polymer | Total (parts by mass) | Photopolymerization initiator | | Coatability | Film strength | Adhesiveness | | Peelability |
|---|---|---|---|---|---|---|---|---|---|---|---|---|---|---|---|---|---|---|---|---|---|---|
| | DAAA | IBXMA | HEAA | MAm | DMAA | DEAA | ACMO | Light ester BC | HEMA | HPMA | IBXA | Acrylic acid | | P-8 | | TPO | Irg 184 | | | External force resistance | Warm water resistance | |
| Example 10 | 10 | 20 | 15 | - | - | - | - | - | - | - | 5 | - | 50 | 50 | 100 | 3.0 | 1.0 | 3 | 3 | 4 | 3 | 3 |
| Example 11 | 10 | 20 | - | 15 | - | - | - | - | - | - | 5 | - | 50 | 50 | 100 | 3.0 | 1.0 | 3 | 3 | 3 | 3 | 4 |
| Example 12 | 10 | 20 | - | - | 15 | - | - | - | - | - | 5 | - | 50 | 50 | 100 | 3.0 | 1.0 | 3 | 3 | 3 | 4 | 3 |
| Example 13 | 10 | - | 10 | 10 | - | - | - | - | - | - | - | 5 | 35 | 65 | 100 | 2.5 | - | 3 | 3 | 4 | 3 | 4 |
| Example 14 | 10 | - | 10 | - | 10 | - | - | - | - | - | - | 5 | 35 | 65 | 100 | 2.5 | - | 3 | 3 | 3 | 4 | 4 |
| Example 15 | 10 | - | 10 | - | - | 10 | - | - | - | - | - | 5 | 35 | 65 | 100 | 2.5 | - | 3 | 3 | 3 | 3 | 3 |
| Example 16 | 10 | - | 10 | - | - | - | 10 | - | - | - | - | 5 | 35 | 65 | 100 | 2.5 | - | 3 | 3 | 4 | 3 | 3 |
| Example 17 | 10 | - | 10 | - | - | - | - | 10 | - | - | - | 5 | 35 | 65 | 100 | 2.5 | - | 3 | 3 | 3 | 3 | 4 |
| Comparative Example 6 | - | 20 | 15 | - | - | - | - | - | - | - | 15 | - | 50 | 50 | 100 | 3.0 | 1.0 | 2 | 2 | 2 | 2 | 2 |
| Comparative Example 7 | - | 20 | - | 15 | - | - | - | - | 10 | - | 5 | - | 50 | 50 | 100 | 3.0 | 1.0 | 1 | - | - | - | - |
| Comparative Example 8 | - | - | 10 | 10 | - | - | - | - | - | 10 | - | 5 | 35 | 65 | 100 | 2.5 | - | 1 | - | - | - | - |

[Table 7]

| | Monomer | | | | Amount of monomers (parts by mass) | Polymer or oligomer | | | | | Total (parts by mass) | Photopolymerization initiator | | Coatability | Film strength | Adhesiveness | | Peelability |
|---|---|---|---|---|---|---|---|---|---|---|---|---|---|---|---|---|---|---|
| | DAAA | IBXMA | HEAA | MAm | | P-8 | P-16 | P-24 | P-25 | U-15HA | | TPO | Irg 184 | | | External force resistance | Warm water resistance | |
| Example 18 | 25 | 30 | 15 | 20 | 90 | 10 | - | - | - | - | 100 | 8.0 | - | 3 | 2 | 3 | 3 | 3 |
| Example 19 | 20 | 20 | 10 | 10 | 60 | - | 40 | - | - | - | 100 | 4.0 | 4.0 | 3 | 3 | 4 | 4 | 4 |
| Example 20 | 20 | 25 | 10 | - | 55 | - | - | 45 | - | - | 100 | 4.0 | 4.0 | 3 | 3 | 4 | 3 | 4 |
| Example 21 | 20 | 25 | 10 | - | 55 | - | - | - | 45 | - | 100 | 4.0 | 4.0 | 3 | 3 | 3 | 3 | 3 |
| Example 22 | 20 | 20 | 10 | 10 | 60 | - | - | 40 | - | - | 100 | 4.2 | - | 3 | 3 | 3 | 3 | 3 |
| Example 23 | 20 | 20 | 10 | 10 | 60 | - | - | - | 40 | - | 100 | 4.2 | - | 3 | 3 | 3 | 2 | 4 |
| Example 24 | 20 | 20 | - | - | 40 | - | - | - | - | 60 | 100 | 4.0 | - | 3 | 3 | 3 | 3 | 3 |

Other abbreviations and manufacturers denoted in Tables 5 to 7 are as follows.

HEAA: hydroxyethyl acrylamide (manufactured by KJ Chemicals Corporation)
MAm: methacrylamide (manufactured by Mitsui Chemicals Inc.)
DMAA: dimethyl acrylamide (manufactured by KJ Chemicals Corporation)
DEAA: diethyl acrylamide (manufactured by KJ Chemicals Corporation)
ACMO: acryloyl morpholine (manufactured by KJ Chemicals Corporation)
LIGHT ESTER BC: butoxy diethylene glycol methacrylate (manufactured by KYOEISHA CHEMICAL Co., LTD)
HPMA: 2-hydroxypropyl methacrylate (manufactured by KYOEISHA CHEMICAL Co., LTD)
IBXA: isobornyl acrylate (manufactured by KYOEISHA CHEMICAL Co., LTD) • Acrylic acid: manufactured by NIPPON SHOKUBAI CO., LTD.
Irg 184: 1-hydroxycyclohexyl phenyl ketone (photopolymerization initiator, IRGACURE 184, manufactured by BASF)
U-15HA: urethane acrylate oligomer (NK OLIGO U-15HA manufactured by SHIN-NAKAMURA CHEMICAL CO., LTD., theoretical molecular weight of 2,300)

(Examples 25 to 28 and Comparative Examples 9 to 11)

Compositions described below were placed in a container provided with a stirring device. Then, the compositions were mixed with each other using the stirring device until the compositions became homogeneous. A nail cosmetic material of Example 25 was obtained.

<Composition of Nail Cosmetic Material of Example 25>

- Diacetone acrylamide (DAAA): 20 parts by mass
- Isobornyl methacrylate (IBXMA): 20 parts by mass
- Hydroxyethyl acrylamide (HEAA): 10 parts by mass
- Methacrylamide (MAm): 10 parts by mass
- Urethane methacrylate polymer (P-8): 40 parts by mass
- 1-Hydroxycyclohexyl phenyl ketone (Irg 184, photopolymerization initiator, IRGACURE 184, manufactured by BASF): 4.0 parts by mass
- Diphenyl(2,4,6-trimethylbenzoyl)phosphine oxide (TPO, photopolymerization initiator, LUCIRIN TPO manufactured by BASF): 4.0 parts by mass

Nail cosmetic materials of Examples 26 to 28 and Comparative Examples 9 to 11 were obtained through the same method as that in the preparation of the nail cosmetic material of Example 25 except that the types and the addition amounts of monomers, polymers, and photopolymerization initiators to be used were changed as shown in Table 8.

<Formation of Base Coat Layer (Base Layer)>

After nails of the hand were coated with each of the obtained nail cosmetic materials using a paint brush, the nail cosmetic materials were photocured (exposed for 60 seconds) using a gel nail exclusive UV device (36 W) to form base coat layers. The thickness of each formed base coat layer was about 120 μm.

<Formation of Gel Nail Layer>

Next, each obtained base coat layer was coated with commercially available CALGEL #CG-03 FRESH PINK (manufactured by MOGA BROOK CO., ltd.) as a color layer using a paint brush, and then, irradiation was performed for 1 minute using an ultraviolet lamp (36 W). Thereafter, the base coat layer was coated with commercially available BIO GEL (manufactured by TAKARA BELMONT Corp.) as a top layer using a paint brush, and irradiation was similarly performed for 2 minutes using the ultraviolet lamp (36W). Each gel nail layer formed was visually observed, and as a result, the gel nail layer was completely cured. The thickness of each film in which the color layer and the top layer were combined was about 250 μm.

[0075] [Table 8]

| | Monomer | | | | | | | | | | | Amount of monomers (parts by mass) | Polymer | | Total (parts by mass) | Photopolymerization initiator | | Coatability | Film strength | Adhesiveness | | Peelability |
|---|---|---|---|---|---|---|---|---|---|---|---|---|---|---|---|---|---|---|---|---|---|---|
| | DAAA | IBXMA | HEAA | MAm | DMAA | ACMO | HEMA | HPMA | IBXA | NVP | Acrylic acid | | P-8 | P-9 | | TPO | Irg 184 | | | External force resistance | Warm water resistance | |
| Example 25 | 20 | 20 | 10 | 10 | - | - | - | - | - | - | - | 60 | 40 | - | 100 | 4.0 | 4.0 | 3 | 3 | 4 | 4 | 4 |
| Example 26 | 10 | 20 | 10 | 5 | - | - | - | - | - | - | - | 45 | 55 | - | 100 | 3.0 | - | 3 | 3 | 4 | 3 | 4 |
| Example 27 | 20 | 20 | 10 | - | - | 10 | - | - | - | - | - | 60 | - | 40 | 100 | 4.0 | - | 3 | 3 | 3 | 4 | 4 |
| Example 28 | 20 | 20 | 10 | - | 10 | - | - | - | - | - | - | 60 | - | 40 | 100 | 4.0 | - | 3 | 3 | 3 | 4 | 4 |
| Comparative Example 9 | - | - | - | - | - | - | 20 | - | 20 | - | 5 | 45 | - | 55 | 100 | 3.6 | - | 3 | 3 | 2 | 1 | 2 |
| Comparative Example 10 | - | - | - | - | - | - | 10 | 10 | 15 | - | 10 | 45 | - | 55 | 100 | 3.6 | - | 3 | 3 | 1 | 2 | 2 |
| Comparative Example 11 | - | - | - | - | - | - | 10 | - | 20 | 10 | 5 | 45 | - | 55 | 100 | 3.6 | - | 3 | 3 | 2 | 2 | 2 |

(Example 29 and Comparative Example 12)

Compositions described below were placed in a container provided with a stirring device. Then, the compositions were mixed with each other using the stirring device until the compositions became homogeneous. Nail cosmetic materials of Example 29 and Comparative Example 12 were obtained.

<Composition of Nail Cosmetic Material of Example 29>

- Diacetone acrylamide: 20 parts by mass
- Isobornyl methacrylate: 20 parts by mass
- Hydroxyethyl acrylamide: 10 parts by mass
- Methacrylamide: 10 parts by mass
- Urethane methacrylate polymer (P-8): 40 parts by mass
- 1-Hydroxycyclohexyl phenyl ketone (photopolymerization initiator, IRGACURE 184, manufactured by BASF): 4.0 parts by mass
- Diphenyl(2,4,6-trimethylbenzoyl)phosphine oxide (photopolymerization initiator, manufactured by BASF, LUCIRIN TPO): 4.0 parts by mass
- 2-Propanol: 10 parts by mass

<Composition of Nail Cosmetic Material of Comparative Example 12>

- 2-Hydroxyethyl methacrylate: 20 parts by mass
- Isobornyl methacrylate: 20 parts by mass
- Hydroxyethyl acrylamide: 10 parts by mass
- Methacrylamide: 10 parts by mass
- Urethane methacrylate polymer (P-8): 40 parts by mass
- 1-Hydroxycyclohexyl phenyl ketone (photopolymerization initiator, IRGACURE 184, manufactured by BASF): 4.0 parts by mass
- Diphenyl(2,4,6-trimethylbenzoyl)phosphine oxide (photopolymerization initiator, LUCIRIN TPO manufactured by BASF): 4.0 parts by mass
- 2-Propanol: 10 parts by mass

<Formation of Base Coat Layer (Base Layer)>

A nail-shaped aluminum substrate was coated with 0.20 g of a nail cosmetic material obtained in Example 29 or Comparative Example 12 using a paint brush, and was irradiated with an ultraviolet lamp (36W) for 2 minutes. In this manner, an aluminum substrate on which a base coat layer was formed was produced.

After washing the surface of the base coat layer with ethanol in order to measure the thickness of the cured film, the formed film was visually observed. As a result, the formed film had been completely cured. In addition, the film thickness at this time was about 80 μm (± 10 μm).

< Formation of Gel Nail Layer>

The aluminum substrate on which the base coat layer was formed was coated with commercially available CALGEL #CG-03 FRESH PINK (manufactured by MOGA BROOK CO., ltd.) as a color layer using a paint brush, and then, irradiation was performed for 1 minute using an ultraviolet lamp (36 W). Thereafter, the base coat layer was coated with commercially available TOP GEL (manufactured by MOGA BROOK CO., ltd.) as a top layer using a paint brush, and irradiation was similarly performed for 2 minutes using the ultraviolet lamp (36W). In this manner, an aluminum substrate 1 which includes the gel nail layer on the base coat layer in which the nail cosmetic material of Example 29 was cured was produced. The gel nail layer formed was visually observed, and as a result, the gel nail layer was completely cured. The thickness of the film in which the color layer and the top layer were combined was about 250 μm.

An aluminum substrate 2 in which the nail cosmetic material of Comparative Example 12 was used in a base coat layer was similarly produced, and the adhesiveness and the peelability were evaluated through the same method as described above. The results are shown in Table 9.

[Table 9]

| | Coatability | Film strength | Adhesiveness | | Peelability |
|---|---|---|---|---|---|
| | | | External force resistance | Warm water resistance | |
| Example 29 | 3 | 3 | 3 | 3 | 4 |
| Comparative Example 12 | 2 | 2 | 2 | 2 | 3 |

## Claims

1. A nail cosmetic material comprising:

   diacetone acrylamide as a component 1; and
   at least one compound selected from the group consisting of isobornyl methacrylate, hydroxyethyl acrylamide, methacrylamide, dimethyl acrylamide, diethyl acrylamide, acryloyl morpholine, and butoxy diethylene glycol methacrylate, as a component 2.

2. The nail cosmetic material according to claim 1, wherein the component 2 includes at least two compounds selected from the group consisting of isobornyl methacrylate, hydroxyethyl acrylamide, methacrylamide, dimethyl acrylamide, diethyl acrylamide, acryloyl morpholine, and butoxy diethylene glycol methacrylate.

3. The nail cosmetic material according to claim 1, wherein the component 2 includes isobornyl methacrylate; and at least one compound selected from the group consisting of hydroxyethyl acrylamide, methacrylamide, and dimethyl acrylamide.

4. The nail cosmetic material according to any one of claims 1 to 3, further comprising:

   a photopolymerization initiator.

5. The nail cosmetic material according to any one of claims 1 to 4, further comprising:

   a polymer and/or an oligomer.

6. The nail cosmetic material according to claim 5, wherein the polymer and/or the oligomer have a urethane bond.

7. The nail cosmetic material according to claim 5 or 6, wherein the polymer and/or the oligomer have a (meth)acrylic group.

8. The nail cosmetic material according to any one of claims 5 to 7, wherein the total content of the polymer and the oligomer is 10 to 60 parts by mass with respect to 100 parts by mass of the nail cosmetic material.

9. The nail cosmetic material according to any one of claims 1 to 8, wherein the content of the component 1 is 1 to 50 parts by mass with respect to 100 parts by mass of the nail cosmetic material.

10. The nail cosmetic material according to any one of claims 1 to 9, wherein the total content of the components 2 is 5 to 80 parts by mass with respect to 100 parts by mass of the nail cosmetic material.

11. The nail cosmetic material according to any one of claims 1 to 10, further comprising:

an organic solvent.

**12.** A nail art kit comprising:

the nail cosmetic material according to any one of claims 1 to 11.

## INTERNATIONAL SEARCH REPORT

International application No.
PCT/JP2016/065441

A. CLASSIFICATION OF SUBJECT MATTER
*A61K8/81*(2006.01)i, *A61Q3/02*(2006.01)i

According to International Patent Classification (IPC) or to both national classification and IPC

B. FIELDS SEARCHED

Minimum documentation searched (classification system followed by classification symbols)
A61K8/81, A61Q3/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | WO 2014/157272 A1 (Fujifilm Corp.), 02 October 2014 (02.10.2014), & US 2015/0352032 A1 & EP 2979687 A1 & CN 105120837 A | 1-12 |
| A | JP 2013-043853 A (Jujo Chemical Co., Ltd.), 04 March 2013 (04.03.2013), (Family: none) | 1-12 |
| A | JP 61-249910 A (Revlon Inc.), 07 November 1986 (07.11.1986), & EP 199325 A2 | 1-12 |
| A | JP 2008-195627 A (Toyo Ink Manufacturing Co., Ltd.), 28 August 2008 (28.08.2008), (Family: none) | 1-12 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 July 2016 (28.07.16) | 09 August 2016 (09.08.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004275736 A
- JP 2002505915 U
- JP 4981184 B